# EUROPÄISCHE PATENTSCHRIFT

(11) **EP 2 687 265 B1**
(45) Veröffentlichungstag und Bekanntmachung des Hinweises auf die Patenterteilung: **18.11.2015**
(21) Anmeldenummer: 13164264.7
(22) Anmeldetag: 18.04.2013
(51) Int. Cl.: A61Q 5/04, A61K 8/41, A61K 8/58, D06M 15/643, A61K 8/898

(54) **Verfahren zur Umformung keratinhaltiger Fasern**
Method for reshaping fibres containing keratin
Procédé de déformation de fibres contenant de la kératine

(30) Priorität: 29.05.2012 DE 102012208959
(43) Veröffentlichungstag der Anmeldung: 22.01.2014
(73) Patentinhaber: Henkel AG&Co. KGAA, 40589 Düsseldorf (DE)
(72) Erfinder: Schulze Zur Wiesche, Erik, 20144 Hamburg (DE); Poppe, Elisabeth, 22763 Hamburg (DE); Noll, Monika, 22851 Norderstedt (DE)

(56) Entgegenhaltungen:
- JP-A- H02 250 814
- US-A- 6 117 420
- US-A1- 2002 187 117
- US-A1- 2007 202 065

## Beschreibung

Die Erfindung betrifft das technische Gebiet der Umformung keratinhaltiger Fasern, insbesondere menschlicher Haare. Gegenstand der Erfindung ist ein verbessertes Verfahren zur Umformung keratinhaltiger Fasern, insbesondere menschlicher Haare. Als keratinhaltige Fasern können prinzipiell alle tierischen Haare, z.B. Wolle, Roßhaar, Angorahaar, Pelze, Federn und daraus gefertigte Produkte oder Textilien eingesetzt werden. Bevorzugt wird die Erfindung jedoch im Rahmen einer Haarumformung, insbesondere der Glättung krauser menschlicher Haare sowie daraus gefertigter Perücken, eingesetzt.

Eine dauerhafte Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann in dem so genannten Fixierschritt mit einer Oxidationsmittelzubereitung behandelt, gespült und nach oder während des Fixierschritts von den Verformungshilfsmitteln (z.B. Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R-S-S-R +HSO₃⁽⁻⁾ → R-SH ⁺R-S-SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glattgekämmt. Anstelle des Wicklers ist es auch möglich, die Faser auf ein Glättungsboard glattzulegen. Glättungsboarde sind üblicherweise rechteckige Tafeln z.B. aus Kunststoff. Vorzugsweise ist die Faser dabei mit der keratinreduzierenden Zubereitung benetzt.

Eine weitere Möglichkeit der Haarglättung ist das Glätten mit einem heißen Eisen. Allerdings verändert sich die Struktur der keratinhaltigen Faser bei der Wärmebehandlung des Haars während des Glättens. Dieser Änderung der Faserstruktur sollte durch geeignete Maßnahmen entgegengewirkt werden.

Die Glättung mittels Glätteisen kann durch vorherige Anwendung alkalischer Produkte unterstützt werden. Solche alkalischen Umformumgsmittel führen im Gegensatz zur Umformung mittels keratinreduzierender und -oxidierender Zusammensetzungen nicht zu einer Umformung der Disulfidbrücken, sondern zu einer Zerstörung der Disulfidbrücken unter Bildung von Monosulfidbrücken. Je nach Konzentration und Anwendungsdauer der alkalischen Umformumgsmittel werden auch Proteinketten hydrolytisch gespalten. Der pH-Wert der alkalischen Umformumgsmittel liegt üblicherweise im Bereich von 11-14, vorzugsweise von 12-13.

Im Allgemeinen haben die bekannten Umformungsverfahren, besonders bei der Glättung, den Nachteil, daß sich die keratinhaltige Faser elektrostatisch auflädt. Darüber hinaus führt die Behandlung mit Umformumgsmitteln zu einer gesteigerten Hydrophilität der Haare, was die Frisierbarkeit erschwert und den Griff, die Kämmbarkeit sowie den Glanz verschlechtert.

Aus US2007/0202065 A1 ist ein derartiges Umformungsverfahren bekannt, welches aminofunktionelle Silikone in einem Vorbehandlungsschritt benutzt

Aufgabe der Erfindung ist es daher, ein Umformungsverfahren für keratinhaltige Fasern, insbesondere für menschliches Haar, bereitzustellen, das ein sehr gutes und dauerhaftes Umformungsergebnis liefert und gleichzeitig die elektrostatische Aufladung sowie Hydrophilierung des Haares minimiert, die Faser pflegt sowie die Struktur der Faser schont.

Überraschenderweise wurde gefunden, daß eine Vorbehandlung der Fasern mit speziellen Vorbehandlungsmitteln bis zu 48 Stunden vor dem Umformungsvorgang die negativen Folgen der Umformung deutlich minimiert und insbesondere die elektrostatische Aufladung sowie Hydrophilierung des Haares minimiert.

Gegenstand der Anmeldung ist ein Verfahren zur Umformung, insbesondere zur Glättung, keratinhaltiger Fasern, insbesondere menschlicher Haare, in welchem
(i) ein Vorbehandlungsmittel, enthaltend aminofunktionelle(s) Silikon(e) mit terminale(r/n) Hydroxygruppe(n) auf die keratinischen Fasern aufgetragen und dort belassen wird
(ii) die Fasern nach einer Einwirkzeit
   - nicht gespült werden,
   - gegebenenfalls getrocknet werden,
(iii) die Fasern mit einem Umformungsmittel behandelt werden,
(iv) die Fasern gegebenenfalls gespült werden,
(v) die Fasern gegebenenfalls unter der Einwirkung von Wärme mechanisch verformt werden.

Im erfindungsgemäßen Verfahren wird zunächst ein Vorbehandlungsmittel, enthaltend aminofunktionelle(s) Silikon(e) gemäss Formel (IV) auf die keratinischen Fasern aufgetragen und dort belassen. Dieser Schritt (i) des erfindungsgemäßen Verfahrens kann bis zu 48 Stunden vor der eigentlichen Umformungsbehandlung erfolgen, die Einwirkzeit in Schritt (ii) beträgt demnach bis zu 48 Stunden. Vorzugsweise liegt die Einwirkzeit in Schritt (ii) des erfindungsgemäßen Verfahrens bei 30 Sekunden bis 6 Stunden, weiter bevorzugt bei 1 bis 60 Minuten, vorzugsweise bei 1,5 bis 45 Minuten, weiter bevorzugt bei 2 bis 30 Minuten und insbesondere bei 3 bis 15 Minuten.

Das in Schritt (i) aufgetragene Vorbehandlungsmittel wird nach der Einwirkzeit nicht ausgespült, sondern verbleibt auf der Faser. Die Faser kann in Schritt (ii) optional getrocknet werden, was bei längeren Einwirkzeiten des Vorbehandlungsmittels durch Lufttrocknung erfolgen kann. Bei kürzeren Applikationszeiten kann das Haar beispielsweise mit einem Handtuch frottiert werden. Nach Abschluß des Frottierschritts bleibt eine fühlbare Restfeuchtigkeit im Haar zurück.

Im nächsten Schritt (iii) des erfindungsgemäßen Verfahrens wird ein Umformungshilfsmittel auf die Fasern aufgetragen. Dieses Mittel kann ebenfalls auf den Fasern verbleiben, es kann aber im optionalen Schritt (iv) auch ausgespült werden. Anschließen werden die Fasern in Schritt (v) mechanisch verformt, was bei Raumtemperatur oder auch unter Anwendung von Wärme geschehen kann.

Die im erfindungsgemäßen Verfahren eingesetzten Vorbehandlungsmittel enthalten neben dem speziellen Silikon It. Formel IV gegebenenfalls noch weitere aminofunktionelle(s) Silikon(e) mit terminale(r/n) Hydroxygruppe(n). Solche Silicone können z.B. durch die Formel

M(RₐQ_{b}SiO_{(4-a-b)/2)x}(R_{c}SiO_{(4-c)/2})_{y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe mit Hydroxyfunktion ist, vorzugsweise Hydroxy-Dimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-,-OCH₂ CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist-N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

In erfindungsgemäßen Verfahren können beispielsweise aminofunktionelle Silikone der Formel (Si-II) eingesetzt werden:

R'ₐG₃₋ₐ-Si(OSiG₂)ₙ-(OSiG_{b}R'_{2-b})ₘ-O-SiG₃₋ₐ-OH (Si-II),

worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃,-CH₂CH₂CH₂CH₃, -O-CH₂CH(CH₃)₂, -CH₂CH(CH₃)₂, -O-CH(CH₃)CH₂CH₃,-CH(CH₃)CH₂CH₃, -O-C(CH₃)₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -Q-N(R")-CH₂-CH₂-N(R")₂
   o -Q-N(R")₂
   o -Q-N⁺(R")₃A⁻
   o -Q-N⁺H(R")₂A⁻
   o -Q-N⁺H₂(R")A⁻
   o -Q-N(R")-CH₂CH₂N⁺R"H₂A⁻,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht, R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

In erfindungsgemäßen Verfahren dieser Ausführungsform können vorzugsweise aminofunktionelle Silikone der Formel (Si-IIa) eingesetzt werden: 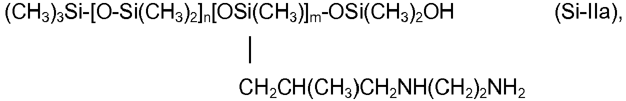 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Besonders bevorzugt sind auch erfindungsgemäße Mittel, die ein aminofunktionelles Silikon der Formel (Si-IIb) 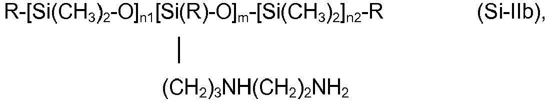 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt. Erfindungsgemäß steht dabei mindestens ein R für -OH.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Die Einsatzmenge des/der aminofunktionellen Silikon(s/e) mit terminale(r/n) Hydroxygruppee(n) in dem im erfindungsgemäßen Verfahren eingesetzten Vorbehandlungsmittel kann variieren, bevorzugte Verfahren sind dadurch gekennzeichnet, daß das Vorbehandlungsmittel bezogen auf sein Gewicht 0,00001 bis 10 Gew.-%, vorzugsweise 0,0001 bis 7,5 Gew.-%, weiter bevorzugt 0,001 bis 5 Gew.-%, noch weiter bevorzugt 0,01 bis 2,5 Gew.-% und insbesondere 0,1 bis 1 Gew.-% aminofunktionelle(s) Silikon(e) mit terminale(r/n) Hydroxygruppee(n) enthält.

Einige spezielle aminofunktionelle(s) Silikon(e) mit terminale(r/n) Hydroxygruppe(n) haben ssich im erfindungsgemäßen Verfahren als besonders geeignet herausgestellt. Diese werden nachstehend beschrieben.

Erfindungsgemäß bevorzugt Verfahren sind dadurch gekennzeichnet, daß im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (I) 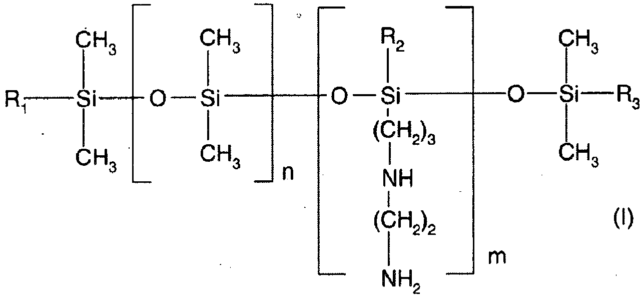 enthält, in der
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind dadurch gekennzeichnet, daß im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (II) 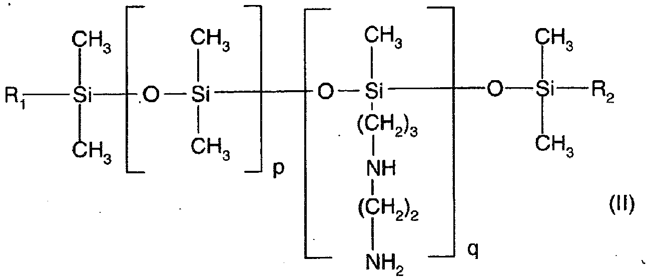 enthält, in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (I) und (II) unterscheiden sich durch die Gruppierung am Si-Atom, das die stickstoffhaltige Gruppe trägt: In Formel (I) bedeutet R2 eine Hydroxygruppe oder eine C1-4-Alkoxygruppe, während der Rest in Formel (II)eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (I) und (II) ist nicht zwingend jedes R1-Si(CH₃)₂-Gruppe an eine -[O-Si(CH₃)₂]-Gruppierung gebunden.

Als besonders wirkungsvoll im Hinblick auf die gewünschten Effekte haben sich im erfindungsgemäßen Verfahren Vorbehandlungsmittel erwiesen, die mindestens ein Silikon der Formel (III) enthalten: 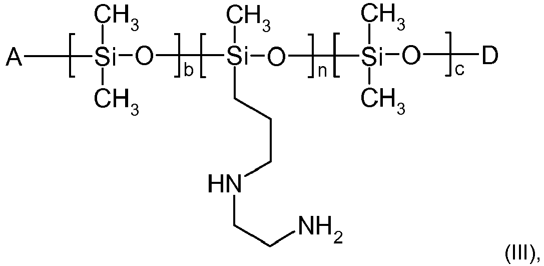 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen, mit den Maßgaben
- n > 0 und b + c > 0
- mindestens eine der Bedingungen A = -OH bzw. D = -H ist erfüllt.

Erfindungsgemäße Verfahren, bei denen im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (III) enthält: 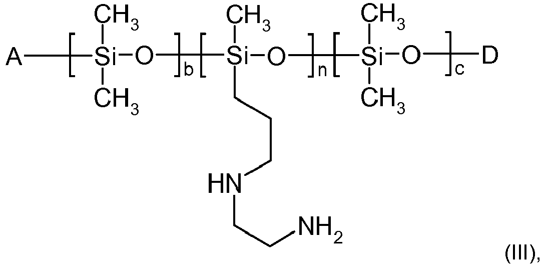 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen, mit den Maßgaben
- n > 0 und b + c > 0
- mindestens eine der Bedingungen A = -OH bzw. D = -H ist erfüllt,
sind demnach erfindungsgemäß bevorzugt. In der vorstehend genannten Formel (III) sind die einzelnen Siloxaneinheiten mit den Indices b, c und n statistisch verteilt, d.h. es muß sich nicht zwingend um Blockcopolymere handeln.

Zwingend enthalten sind in den erfindungsgemässen Vorbehandlungsmitteln die in (IV) gezeigten Silikone, diese sind 4-morpholinomethyl-substituiert. Erfindungsgemäße Verfahren, bei denen das Vorbehandlungsmittel bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines 4-morpholinomethyl-substituierten Silikons der Formel (IV) enthält, 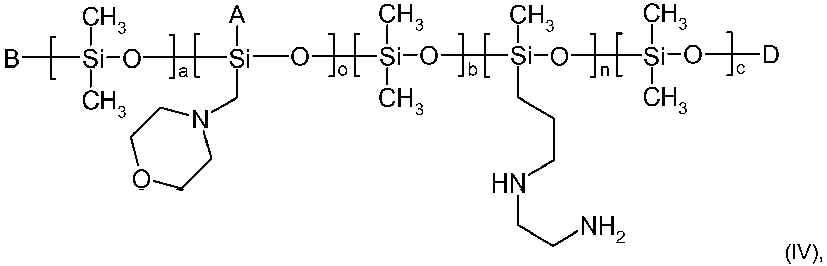 in der
- A: für eine über ein -O- gebundene Struktureinheit (i), (ii) oder (iii) 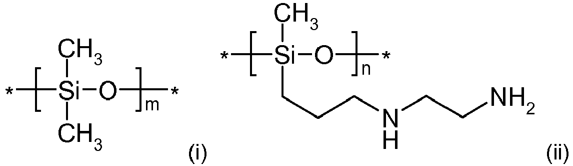 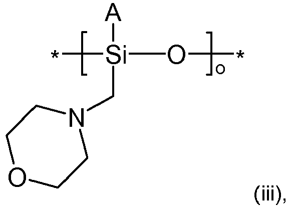 oder einen über ein -O- gebundenen oligomeren oder polymeren Rest enthaltend Struktureinheiten der Formeln (I), (II)oder (III) oder die Hälfte eines verbindenden O-Atoms zu einer Struktureinheit (III) oder für-OH steht,
- *: für eine Bindung zu einer der Struktureinheiten (i), (ii) oder (iii) oder für eine Endgruppe B (Si-gebunden) oder D (O-gebunden) steht,
- B: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- a, b und: c für ganze Zahlen zwischen 0 und 1000 stehen, mit der Maßgabe a + b + c > 0
- m, n und: o für ganze Zahlen zwischen 1 und 1000 stehen.
mit der Maßgabe, daß mindestens eine der Bedingungen B = -OH bzw. D = -H erfüllt ist,
sind besonders bevorzugt.

Strukturformel (IV) soll verdeutlichen, daß die Siloxangruppen n und o nicht zwingend direkt an eine Endgruppierung B bzw. D gebunden sein müssen. Vielmehr gilt in bevorzugten Formeln (IV) a > 0 oder b > 0 und in besonders bevorzugten Formeln (IV) a > 0 und b > 0, d.h. die terminale Gruppierung B bzw. D ist vorzugsweise an eine Dimethylsiloxy-Gruppierung gebunden. Auch in Formel (IV) sind die Siloxaneinheiten a, b, c, n und o vorzugsweise statistisch verteilt.

Die durch Formel (IV) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, insbesondere zu einem gravierend verbesserten Schutz bei oxidativer Behandlung.

Auch in Formel (IV) kann der Rest A
- für eine über ein -O- gebundene Struktureinheit (i), (ii) oder (iii) oder
- einen über ein -O- gebundenen oligomeren oder polymeren Rest enthaltend Struktureinheiten der Formeln (i), (ii) oder (iii)
- oder die Hälfte eines verbindenden O-Atoms zu einer Struktureinheit (iii) oder für -OH stehen.

Damit wird Formel (IV) präzisiert zu einer der Formeln (IVa), (IVb), (IVc), (IVd), (IVe) oder (IVf):

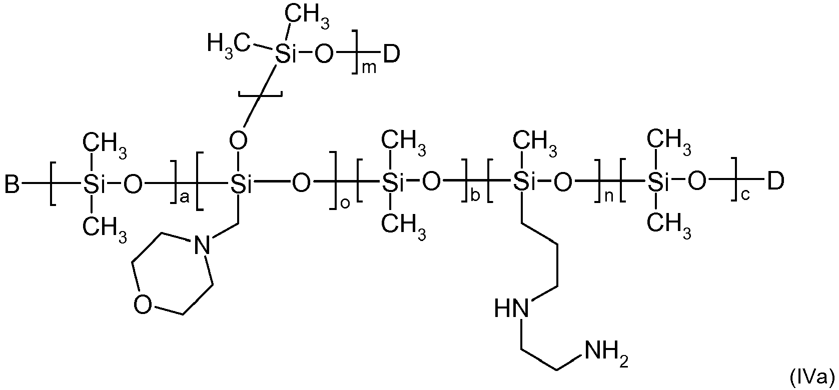

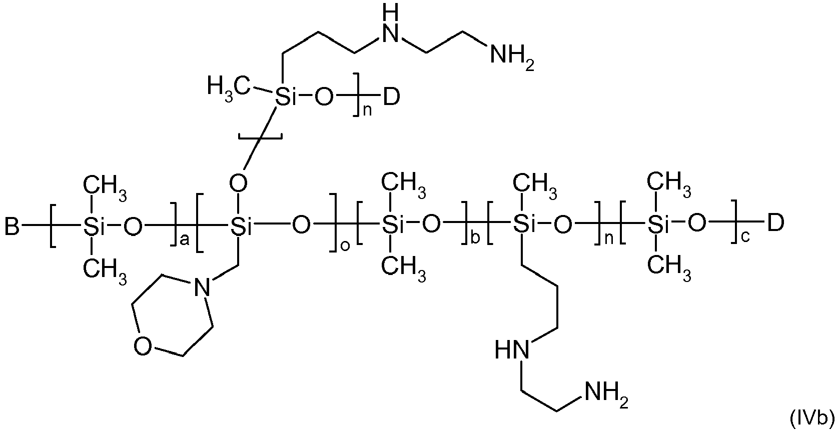

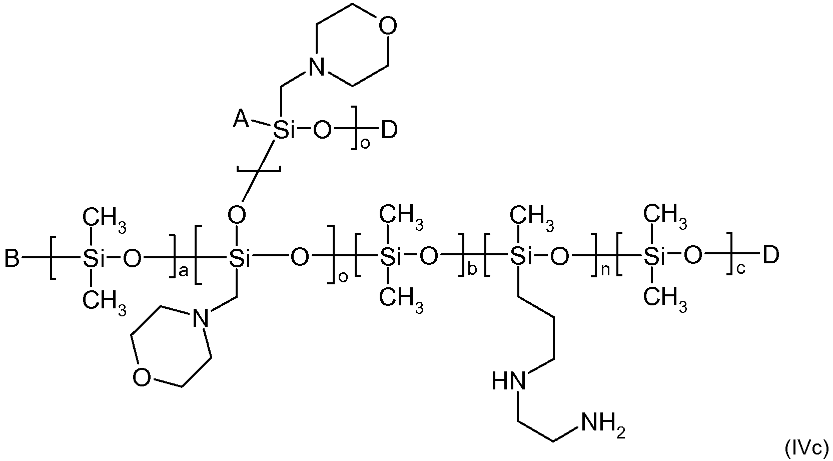

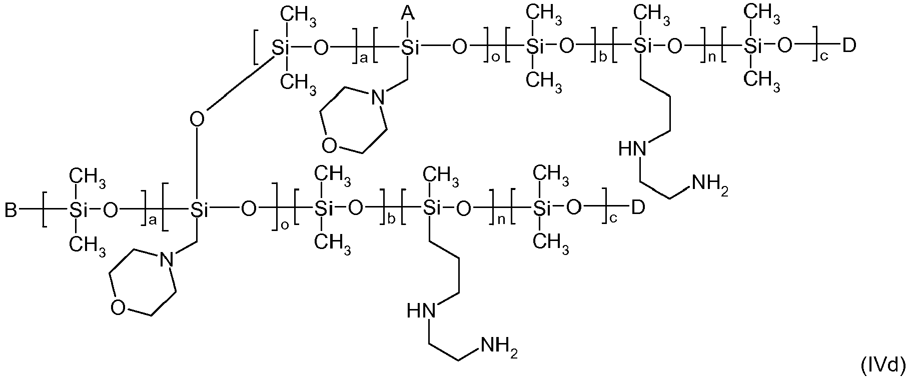

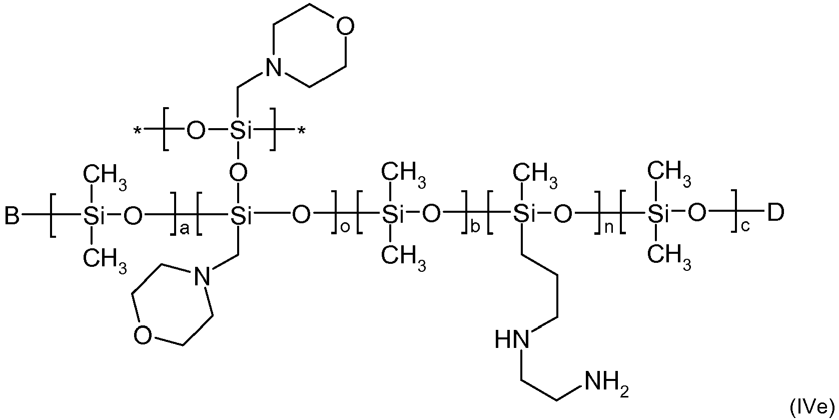

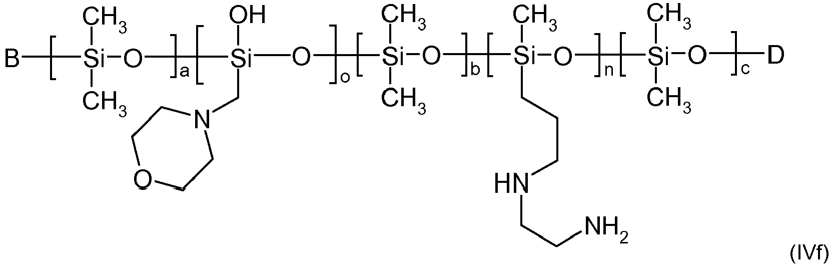

Die Struktureinheit (iii) bzw. die Siloxaneinheiten o in den Formeln (IV) können über die Gruppe A Nest- bzw. Teilkäfigstrukturen ausbilden, wenn A für die Hälfte eines verbindenden O-Atoms zu einer Struktureinheit (III) steht. Erfindungsgemäße Vorbehandlungsmittel, die Silikone mit entsprechenden 4-morpholinomethyl-substituierten Silsesquioxan-Teilstrukturen beinhalten, sind erfindungsgemäß bevorzugt, da diese Silikone zu enorm verbessertem Haarschutz vor oxidativer Behandlung führen.

Es ist in erfindungsgemäßen Verfahren der vorstehend genannten Ausführunmgsform besonders bevorzugt, wenn das Vorbehandlungsmittel es - bezogen auf sein Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% 4-morpholinomethyl-substituierte(s) Silikon(e) enthält.

Unabhängig von der Art des bzw. der eingesetzten aminofunktionellen Silikon(s/e) mit terminale(r/n) Hydroxygruppee(n) enthalten die im erfindungsgemäßen Verfahren eingesetzten Vorbehandlungsmittel das/die Silikon(e) vorzugsweise in Form einer Emulsion, besonders bevorzugt in Form einer Mikroemulsion. Als besonders bevorzugt haben sich Mikroemulsionen erwiesen, die Fettalkohole als Emulgatoren bzw. Stabilisatoren enthalten so daß bevorzugte erfindungsgemäße Verfahren dadurch gekennzeichnet sind, daß das Vorbehandlungsmittel in Form einer Mikroemulsion vorliegt, welche Fettalkohol(e) enthält.

Es hat sich gezeigt, daß die Wirkung der im Rahmen des erfindungsgemäßen Verfahrens in den Vorbehandlungsmitteln eingesetzten Silikone noch gesteigert werden kann, wenn bestimmte nichtionische Komponenten ebenfalls in den Vorbehandlungsmitteln eingesetzt werden. Zudem haben diese nichtionischen Komponenten positive Effekte auf die Lagerstabilität der Vorbehandlungsmittel. Nichtionische Komponenten, die hier besonders geeignet sind, sind Ethoxylate von Decanol, Undecanol, Dodecanol, Tridecanol usw.. Als besonders geeignet haben sich ethoxylierte Tridecanole erwiesen, die mit besonderem Vorzug in die im erfindungsgemäßen Verfahren eingesetzten Vorbehandlungsmittel inkorporiert werden. Erfindungsgemäß besonders bevorzugte Verfahren sind dadurch gekennzeichnet, daß das Vorbehandlungsmittel- bezogen auf sein Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen enthält.

Die im erfindungsgemäßen Verfahren eingesetzten Vorbehandlungsmittel können darüber hinaus weitere übliche Inhaltsstoffe kosmetischer Mittel enthalten.

Wie bereits erwähnt, kann die Faser in Schritt (ii) optional getrocknet werden, was bei längeren Einwirkzeiten des Vorbehandlungsmittels durch Lufttrocknung erfolgen kann. Bei kürzeren Applikationszeiten kann das Haar beispielsweise mit einem Handtuch frottiert werden. Nach Abschluß des Frottierschritts bleibt eine fühlbare Restfeuchtigkeit im Haar zurück. Erfindungsgemäß bevrozugte Verfahren sind dadurch gekennzeichnet, daß die Fasern in Schritt (ii) getrocknet werden.

Im nächsten Schritt (iii) des erfindungsgemäßen Verfahrens wird ein Umformungshilfsmittel auf die Fasern aufgetragen. Dieses Mittel kann ebenfalls auf den Fasern verbleiben, es kann aber im optionalen Schritt (iv) auch ausgespült werden. Anschließen werden die Fasern in Schritt (v) mechanisch verformt, was bei Raumtemperatur oder auch unter Anwendung von Wärme geschehen kann.

Eine bevorzugte Variante des erfindungsgemäßen Verfahrens ist dadurch gekennzeichnet, daß die Fasern in Schritt (iii) einer Behandlung mit einem alkalischen Mittel unterworfen werden. Der pH-Wert der alkalischen Umformumgsmittel liegt üblicherweise im Bereich von 11-14, vorzugsweise von 12-13.

Die zur Einstellung des bevorzugten pH-Wertes erfindungsgemäß verwendbaren Alkalisierungsmittel können aus der Gruppe Ammoniak, basischen Aminosäuren, Alkalihydroxiden, Alkanolaminen, Alkalimetallmetasilikaten, Alkaliphosphaten und Alkalihydrogenphosphaten ausgewählt werden. Als Alkalimetallionen dienen bevorzugt Lithium, Natrium, Kalium, insbesondere Natrium oder Kalium.

Die als Alkalisierungsmittel einsetzbaren basischen Aminosäuren werden bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide werden bevorzugt ausgewählt aus der Gruppe Natriumhydroxid und Kaliumhydroxid.

Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-l-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Ganz besonders bevorzugte erfindungsgemäße Verfahren sind dadurch gekennzeichnet, daß die Fasern in Schritt (iii) einer Behandlung mit einem alkalischen Mittel unterworfen werden, welches - bezogen auf sein Gewicht - 1 bis 5 Gew.-%, vorzugsweise 1,5 bis 4,5 Gew.-%, weiter bevorzugt 2 bis 4 Gew.-% und insbesondere 2,5 bis 3,5 Gew.-% Natriumhydroxid enthält.

Weitere besonders bevorzugte erfindungsgemäße Verfahren sind dadurch gekennzeichnet, daß die Fasern in Schritt (iii) einer Behandlung mit einem alkalischen Mittel unterworfen werden, welches - bezogen auf sein Gewicht - 0,5 bis 2,5 Gew.-%, vorzugsweise 0,75 bis 2,25 Gew.-%, weiter bevorzugt 1 bis 2 Gew.-% und insbesondere 1,25 bis 1,75 Gew.-% Calciumhydroxid enthält.

Weitere besonders bevorzugte erfindungsgemäße Verfahren sind dadurch gekennzeichnet, daß die Fasern in Schritt (iii) einer Behandlung mit einem alkalischen Mittel unterworfen werden, welches - bezogen auf sein Gewicht - 0,5 bis 2,5 Gew.-%, vorzugsweise 0,75 bis 2,25 Gew.-%, weiter bevorzugt 1 bis 2 Gew.-% und insbesondere 1,25 bis 1,75 Gew.-% Guanidincarbonat enthält.

Insbesondere bevorzugte erfindungsgemäße Verfahren sind dadurch gekennzeichnet, daß die Fasern in Schritt (iii) einer Behandlung mit einem alkalischen Mittel unterworfen werden, welches - bezogen auf sein Gewicht - 0,5 bis 2,5 Gew.-%, vorzugsweise 0,75 bis 2,25 Gew.-%, weiter bevorzugt 1 bis 2 Gew.-% und insbesondere 1,25 bis 1,75 Gew.-% Calciumhydroxid und 0,5 bis 2,5 Gew.-%, vorzugsweise 0,75 bis 2,25 Gew.-%, weiter bevorzugt 1 bis 2 Gew.-% und insbesondere 1,25 bis 1,75 Gew.-% Guanidincarbonat enthält.

Anschließend an die Behandlung mit einem alkalischen Mittel können die Fasern optional ausgespült {Schritt (iv) des erfindungsgemäßen Verfahrens} und anschließend verformt {Schritt (v) des erfindungsgemäßen Verfahrens} werden. Die Verformung in Schritt (v) kann durch Wärme unterstützt werden.

Alternativ zur Behandlung mit alkalischen Umformungshilfsmitteln können die keratinischen Fasern in Schritt (iii) auch einer Behandlung mit Keratin reduzierenden Substanzen unterworfen werden, wie sie beispielsweise im Rahmen des Dauerwellprozesses Verwendung finden.

Eine weitere erfindungsgemäß bevorzugte Verfahrensvariante ist dadurch gekennzeichnet, daß die Fasern in Schritt (iii) einer Behandlung mit einer keratinreduzierenden Substanz, der Verformung der Faser und der nachfolgenden Behandlung mit einer oxidierenden Substanz unterworfen werden.

Die im ersten Schritt dieses Schrittes (iii) des erfindungsgemäßen Verfahrens verwendbaren Keratin reduzierenden Substanzen (Reduktionsmittel) werden vorzugsweise ausgewählt aus Keratin reduzierenden Verbindungen, insbesondere Verbindungen mit mindestens einer Thiolgruppe sowie deren Derivaten, sowie aus Sulfiten, Hydrogensulfiten und Disulfiten. Verbindungen mit mindestens einer Thiolgruppe sowie deren Derivate sind beispielsweise Thioglykolsäure, Thiomilchsäure, Thioäpfelsäure, Phenylthioglykolsäure, Mercaptoethansulfonsäure sowie deren Salze und Ester (wie z.B. Isooctylthioglycolat und Isopropylthioglycolat), Cysteamin, Cystein, Bunte-Salze und Salze der schwefligen Säure. Bevorzugt geeignet sind die Monoethanolammoniumsalze oder Ammoniumsalze der Thioglykolsäure und/oder der Thiomilchsäure sowie die jeweiligen freien Säuren. Diese werden im Rahmen des erfindungsgemäßen Verfahrens bevorzugt in Form von Zusammensetzungen eingesetzt, welche Konzentrationen von 0,5 bis 2,0 mol/kg dieser Verbindungen enthalten und einen pH-Wert von 5 bis 12, insbesondere von 7 bis 9,5, aufweisen. Zur Einstellung dieser pH-Werte werden vorzugsweise Alkalisierungsmittel wie Ammoniak, Alkali- und Ammoniumcarbonate und -hydrogencarbonate oder organische Amine wie Monoethanolamin verwendet.
Beispiele für keratinreduzierende Verbindungen vom Typ der Disulfite sind Alkalidisulfite, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele fuer keratinreduzierende Verbindungen vom Typ der Hydrogensulfite sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen vom Typ der Sulfite sind Sulfite als Alkali-, Ammonium- oder Alkanolammonium-Salz auf Basis eines C₂-C₄-Mono-, Di- oder Trialkanolamins. Ammoniumsulfit ist dabei bevorzugt. Die Verwendung von Sulfit und/oder Disulfit und/oder Hydrogensulft erfolgt im Rahmen dieses Schrittes (iii) des erfindungsgemäßen Verfahrens bevorzugt bei pH 5 bis 8, insbesondere von pH 6 bis 7,5. Bevorzugte C₂-C₄-Alkanolamine sind erfindungsgemäß 2-Aminoethanol (Monoethanolamin) und N,N,N-Tris(2-hydroxyethyl)amin (Triethanolamin). Monoethanolamin ist ein besonders bevorzugtes C₂-C₄-Alkanolamin, das im Rahmen des erfindungsgemäßen Verfahrens bevorzugt in Form von Zusammensetzungen eingesetzt wird, welche eine Konzentrationen von 0.2 bis 6 Gew.-% dieses Amins, bezogen auf die gesamte Zusammensetzung, aufweisen.

Erfindungsgemäß besonders bevorzugte Reduktionsmittel sind Thioglykolsäure und Thiomilchsäure sowie deren Salze.

Im Rahmen dieses Schrittes (iii) des erfindungsgemäßen Verfahrens wird das Reduktionsmittel bevorzugt in Form einer Zusammensetzung eingesetzt, die das Reduktionsmittel in einer Menge von 5 bis 20 Gew.-%, bezogen auf die gesamte Zusammensetzung, enthält.
Darüber hinaus kann die Lösung des Reduktionsmittels weitere Komponenten enthalten, die die Wirkung auf das Keratin fördern. Solche Komponenten sind z.B. Quellmittel wie z.B. niedere Alkohole und wasserlösliche Glycole oder Polyole wie z.B. Glycerin, 1,2-Propylenglycol oder Sorbit und Harnstoff oder Harnstoffderivate wie z.B. Allantoin und Guanidin sowie Imidazol und dessen Derivate. Bevorzugte weitere Komponenten sind 1,2-Propylenglycol, insbesondere in einer Menge von 5-20 Gew.-%, sowie Harnstoff, insbesondere in einer Menge von 1-10 Gew.-%. Die Mengenangaben beziehen sich jeweils auf die gesamte Lösung bzw. Zusammensetzung, die die Keratin reduzierende Substanz enthält. In einer bevorzugten Ausführung enthält die Zusammensetzung, die die Keratin reduzierende Substanz enthält 5-20 Gew.-% 1,2-Propylenglycol und/oder 1-10 Gew.-% Harnstoff.
Weiterhin kann die Zusammensetzung, die die Keratin reduzierende Substanz enthält oberflächenaktive Stoffe, insbesondere solche aus der Gruppe der anionischen, amphoteren, zwitterionischen und nichtionischen Tenside enthalten. Diese Tenside haben die Aufgabe, die Benetzung der Keratinoberfläche durch die Behandlungslösung zu fördern, vor allem aber eventuell vorhandene Duftstoffe zu solubilisieren oder stabil zu emulgieren.

Die Temperatur beim In-Kontakt-Bringen des Reduktionsmittels mit dem Haar liegt vorzugsweise in einem Bereich von etwa 10 bis etwa 60°C.
Nach mindestens anteiliger Aufspaltung der Disulfidbrücken und damit nach einer Einwirkzeit, die von der Konzentration und der Temperatur abhängt und üblicherweise eine bis 45 Minuten, vorzugsweise 5 bis 40 Minuten und insbesondere 10 bis 30 Minuten beträgt, wird die wäßrige Zubereitung einer Keratin reduzierenden Substanz ausgespült. Dieses Ausspülen erfolgt vorzugsweise mit warmem Wasser ohne weitere Zusätze und entspricht Schritt (iv) des erfindungsgemäßen Verfahrens.

Danach kann das Haar mit Hilfe geeigneter Umformungshilfsmittel in die gewünschte Form gebracht werden, wobei sowohl glatte Haare dauergewellt als auch krause Haare geglättet werden können. Dieser Schritt (v) des erfindungsgemäßen Verfahrens kann auch durch Wärmeanwendung unterstützt werden.

Anschließend wird eine wäßrige Zubereitung eines Oxidationsmittels aufgebracht, um die geöffneten Disulfidbrücken wieder oxidativ zu schließen. Je nach Art und Menge des Oxidationsmittels kann die Fixierlösung nach der Verformung ausgespült werden, es ist aber auch möglich, diese im Haar zu belassen.

Zur Oxidation der vorhab mindestens anteilsweise gelösten Disulfidbindungen enthalten die wäßrigen Zubereitungen Oxidationsmittel wie z.B. Natriumbromat, Kaliumbromat und/oder Wasserstoffperoxid, gegebenenfalls mit den zur Stabilisierung wäßriger Wasserstoffperoxidzubereitungen üblichen Stabilisatoren. Der pH-Wert solcher wäßriger H₂0₂-Zubereitungen, die üblicherweise etwa 0,5 bis 3,0 Gew.-% H₂0₂ enthalten, liegt bevorzugt bei 2 bis 6. Fixiermittel auf Bromat-Basis enthalten die Bromate üblicherweise in Konzentrationen von 1 bis 10 Gew.-% und der pH-Wert der Lösungen wird auf 4 bis 7 eingestellt.

Oft werden die in diesem Schritt (iii) des erfindungsgemäßen Verfahrens eingesetzten Fixiermittel als Feststoffe formuliert. Sie enthalten das Oxidationsmittel dann in Form eines Festkörpers, z.B. Natriumperborat. Erst kurz vor der Anwendung werden diese Mittel dann mit Wasser versetzt. Ebenfalls möglich und bevorzugt ist, das Oxidationsmittel als 2-Komponenten-System zu formulieren. Die beiden Komponenten, von denen die eine bevorzugt eine schwach saure Wasserstoffperoxidlösung oder eine wäßrige Lösung eines anderen Oxidationsmittels ist und die andere die übrigen Bestandteile enthält, werden ebenfalls erst kurz vor der Anwendung zu einer gebrauchsfertigen Fixierlösung mit einem pH-Wert von 7-9 vermischt.

Erfindungsgemäß bevorzugte Verfahren sind dadurch gekennzeichnet, daß die wäßrige Zubereitung des Oxidationsmittels dieses Schrittes (iii) - bezogen auf ihr Gewicht - 0,1 bis 20 Gew.-%, vorzugsweise 0,5 bis 15 Gew.-% und insbesondere 1,0 bis 10 Gew.-% Wasserstoffperoxid (berechnet als 100%iges H₂O₂) enthält.

Weiter bevorzugte erfindungsgemäße Verfahren sind dadurch gekennzeichnet, daß die wäßrige Zubereitung des Oxidationsmittels dieses Schrittes (iii) - bezogen auf ihr Gewicht - 0,1 bis 15 Gew.-%, vorzugsweise 0,5 bis 12,5 Gew.-%, besonders bevorzugt 0,75 bis 10 Gew.-% und insbesondere 1,0 bis 8 Gew.-% Bromat(e), vorzugsweise Natriumbromat und/oder Kaliumbromat enthält.

Zusätzlich zu den genannten Aktivstoffen können sowohl die Welllösung als auch die Fixierlösung weitere Inhaltsstoffe enthalten.

Unabhängig davon, ob die Haare in Schritt (iii) des erfindungsgemäßen Verfahrens mittels alkalischer oder Keratin reduzierender Umformungshilfsmittel behandelt werden, kann die in Schritt (v) vorgesehen Umformung der Keratinfasern optional durch Wärme unterstützt werden, beispielsweise durch beheizte Lockenwickler oder - besonders bevorzugt - durch Anwendung eine Glätteisens.

Erfindungsgemäße Verfahren, bei denen die Fasern in Schritt (v) einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen werden, sind erfindungsgemäß bevorzugt.

Zusammenfassend sind folgende erfindungsgemäße Verfahrensvarianten besonders bevorzugt:
- Verfahren zur Umformung, insbesondere zur Glättung, keratinhaltiger Fasern, insbesondere menschlicher Haare, in welchem
   (i) ein Vorbehandlungsmittel, enthaltend bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines aminofunktionellen Silikons der Formel (IV) auf die keratinischen Fasern aufgetragen und dort belassen wird
   (ii) die Fasern nach einer Einwirkzeit von 30 Sekunden bis 15 Minuten
      - nicht gespült werden,
      - gegebenenfalls getrocknet werden,
   (iii) die Fasern mit einem Umformungsmittel behandelt werden, welches bezogen auf sein Gewicht 1 bis 5 Gew.-%, vorzugsweise 1,5 bis 4,5 Gew.-%, weiter bevorzugt 2 bis 4 Gew.-% und insbesondere 2,5 bis 3,5 Gew.-% Natriumhydroxid enthält,
   (iv) die Fasern gespült werden,
   (v) die Fasern einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen und mechanisch verformt werden.
- Verfahren zur Umformung, insbesondere zur Glättung, keratinhaltiger Fasern, insbesondere menschlicher Haare, in welchem
   (i) ein Vorbehandlungsmittel, enthaltend bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines aminofunktionellen Silikons der Formel (IV) auf die keratinischen Fasern aufgetragen und dort belassen wird
   (ii) die Fasern nach einer Einwirkzeit von 30 Sekunden bis 15 Minuten
      - nicht gespült werden,
      - gegebenenfalls getrocknet werden,
   (iii) die Fasern mit einem Umformungsmittel behandelt werden, welches bezogen auf sein Gewicht 1 bis 5 Gew.-%, vorzugsweise 1,5 bis 4,5 Gew.-%, weiter bevorzugt 2 bis 4 Gew.-% und insbesondere 2,5 bis 3,5 Gew.-% Calciumhydroxid enthält,
   (iv) die Fasern gespült werden,
   (v) die Fasern einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen und mechanisch verformt werden.
- Verfahren zur Umformung, insbesondere zur Glättung, keratinhaltiger Fasern, insbesondere menschlicher Haare, in welchem
   (i) ein Vorbehandlungsmittel, enthaltend bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines aminofunktionellen Silikons der Formel (IV) auf die keratinischen Fasern aufgetragen und dort belassen wird
   (ii) die Fasern nach einer Einwirkzeit von 30 Sekunden bis 15 Minuten
      - nicht gespült werden,
      - gegebenenfalls getrocknet werden,
   (iii) die Fasern mit einem Umformungsmittel behandelt werden, welches bezogen auf sein Gewicht 1 bis 5 Gew.-%, vorzugsweise 1,5 bis 4,5 Gew.-%, weiter bevorzugt 2 bis 4 Gew.-% und insbesondere 2,5 bis 3,5 Gew.-% Guanidincarbonat enthält,
   (iv) die Fasern gespült werden,
   (v) die Fasern einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen und mechanisch verformt werden.
- Verfahren zur Umformung, insbesondere zur Glättung, keratinhaltiger Fasern, insbesondere menschlicher Haare, in welchem
   (i) ein Vorbehandlungsmittel, enthaltend bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines aminofunktionellen Silikons der Formel (IV) auf die keratinischen Fasern aufgetragen und dort belassen wird
   (ii) die Fasern nach einer Einwirkzeit von 30 Sekunden bis 15 Minuten
      - nicht gespült werden,
      - gegebenenfalls getrocknet werden,
   (iii) die Fasern mit einem Umformungsmittel behandelt werden, welches bezogen auf sein Gewicht 1 bis 5 Gew.-%, vorzugsweise 1,5 bis 4,5 Gew.-%, weiter bevorzugt 2 bis 4 Gew.-% und insbesondere 2,5 bis 3,5 Gew.-% Calciumhydroxid und 0,5 bis 2,5 Gew.-%, vorzugsweise 0,75 bis 2,25 Gew.-%, weiter bevorzugt 1 bis 2 Gew.-% und insbesondere 1,25 bis 1,75 Gew.-% Guanidincarbonat enthält,
   (iv) die Fasern gespült werden,
   (v) die Fasern einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen und mechanisch verformt werden.
- Verfahren zur Umformung, insbesondere zur Glättung, keratinhaltiger Fasern, insbesondere menschlicher Haare, in welchem
   (i) ein Vorbehandlungsmittel, enthaltend bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines aminofunktionellen Silikons der Formel (IV) auf die keratinischen Fasern aufgetragen und dort belassen wird
   (ii) die Fasern nach einer Einwirkzeit von 30 Sekunden bis 15 Minuten
      - nicht gespült werden,
      - gegebenenfalls getrocknet werden,
   (iii) die Fasern mit einem Umformungsmittel behandelt werden, welches 0,5 bis 2,0 mol/kg Thioglykolsäure und/oder Thiomilchsäure enthält und einen pH-Wert von 5 bis 12, insbesondere von 7 bis 9,5, aufweist,
   (iv) die Fasern gespült werden,
   (v) die Fasern mechanisch verformt werden,
   (vi) die Fasern mit einem Fixiermittel behandelt werden, welches- bezogen auf sein Gewicht-0,1 bis 20 Gew.-%, vorzugsweise 0,5 bis 15 Gew.-% und insbesondere 1,0 bis 10 Gew.-% Wasserstoffperoxid (berechnet als 100%iges H₂O₂) enthält.
- Verfahren zur Umformung, insbesondere zur Glättung, keratinhaltiger Fasern, insbesondere menschlicher Haare, in welchem
   (i) ein Vorbehandlungsmittel, enthaltend bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines aminofunktionellen Silikons der Formel (IV) auf die keratinischen Fasern aufgetragen und dort belassen wird
   (ii) die Fasern nach einer Einwirkzeit von 30 Sekunden bis 15 Minuten
      - nicht gespült werden,
      - gegebenenfalls getrocknet werden,
   (iii) die Fasern mit einem Umformungsmittel behandelt werden, welches 0,5 bis 2,0 mol/kg Thioglykolsäure und/oder Thiomilchsäure enthält und einen pH-Wert von 5 bis 12, insbesondere von 7 bis 9,5, aufweist,
   (iv) die Fasern gespült werden,
   (v) die Fasern mechanisch verformt werden,
   (vi) die Fasern mit einem Fixiermittel behandelt werden, welches- bezogen auf sein Gewicht - 0,1 bis 15 Gew.-%, vorzugsweise 0,5 bis 12,5 Gew.-%, besonders bevorzugt 0,75 bis 10 Gew.-% und insbesondere 1,0 bis 8 Gew.-% Bromat(e), vorzugsweise Natriumbromat und/oder Kaliumbromat enthält.

Für bevorzugte Ausführungsformen der erfindungsgemäßen Verwendungen gilt mutatis mutanmdis das zu den erfindungsgemäßen Verfahren Gesagte.

Die folgenden Beispiele, welche ein aminofunktionelles silikon laut der Formel IV enthalten, erläutern den Gegenstand der Erfindung näher.

### Beispiele:

### Alle Angaben in Gew.-%.

### a) Rezepturen

### a1) Vorbehandlungsmittel:

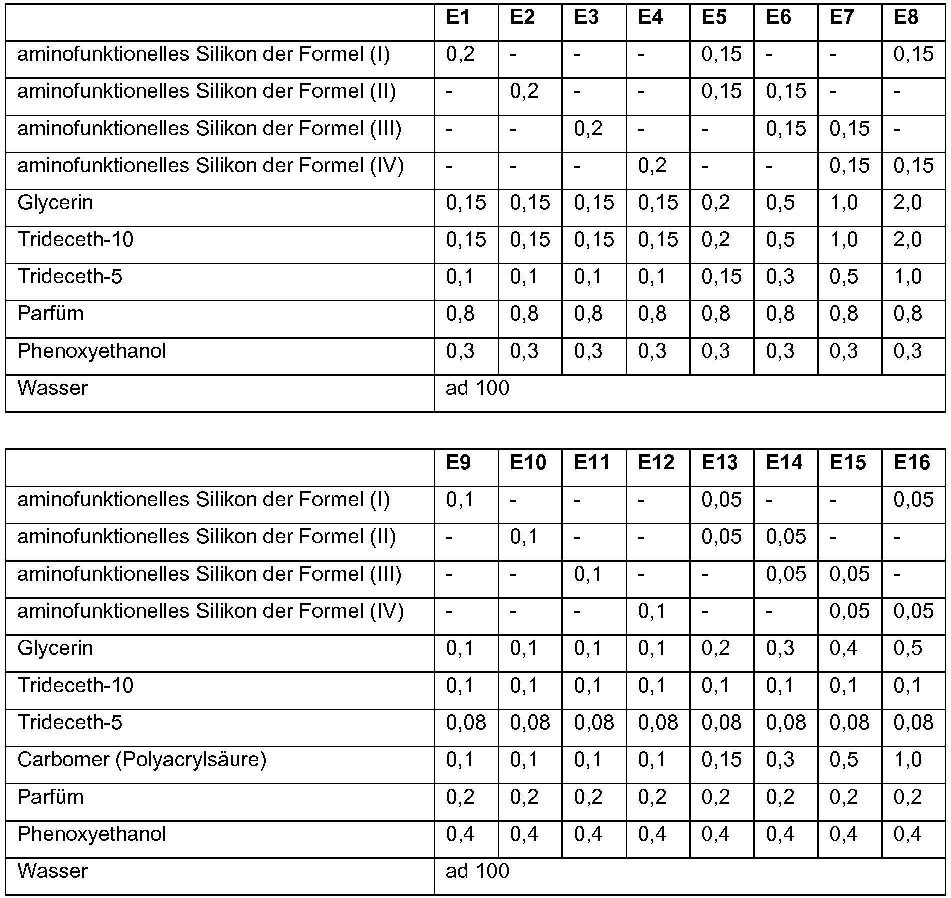

### a2) Glättungsmittel (alkalische "lye-relaxer")

| | G1 | G2 | G3 | G4 | G5 | G6 | G7 | G8 |
|---|---|---|---|---|---|---|---|---|
| Petrolatum | 30 | 30 | 30 | 30 | 30 | 30 | 30 | 30 |
| Paraffinum liquidum | 20 | 20 | 20 | 20 | 20 | 20 | 20 | 20 |
| Cetylakohol | 6 | 6 | 6 | 6 | 6 | 6 | 6 | 6 |
| Stearate-20 | 5 | 5 | 5 | 5 | 5 | 5 | 5 | 5 |
| PEG-60 | 4 | 4 | 4 | 4 | 4 | 4 | 4 | 4 |
| Sodium Hydroxide | 2,0 | 2,25 | 2,5 | 2,75 | 3,0 | 3,25 | 3,5 | 3,75 |
| Wasser | ad 100 | | | | | | | |

### a3) Glättungsmittel (alkalische "non-lye-relaxer")

| 1. relaxer Cremes | | | | | | | | |
|---|---|---|---|---|---|---|---|---|
| | **G9** | **G10** | **G11** | **G12** | **G13** | **G14** | **G15** | **G16** |
| Petrolatum | 30 | 30 | 30 | 30 | 30 | 30 | 30 | 30 |
| Paraffinum liquidum | 20 | 20 | 20 | 20 | 20 | 20 | 20 | 20 |
| Cetearylakohol | 5 | 5 | 5 | 5 | 5 | 5 | 5 | 5 |
| Behentrimonium Methosulfate | 2 | 2 | 2 | 2 | 2 | 2 | 2 | 2 |
| PEG-75 Lanolin | 2 | 2 | 2 | 2 | 2 | 2 | 2 | 2 |
| Cocoamidopropylbetain | 1 | 1 | 1 | 1 | 1 | 1 | 1 | 1 |
| Guanidincarbonat | 2,0 | 2,25 | 2,5 | 2,75 | 3,0 | 3,25 | 3,5 | 3,75 |
| Wasser | ad 100 | | | | | | | |
| | | | | | | | | |

| 2. Aktivatoren | | | | | | | | |
|---|---|---|---|---|---|---|---|---|
| | **G17** | **G18** | **G19** | **G20** | **G21** | **G22** | **G23** | **G24** |
| Silica | 2 | 2 | 2 | 2 | 2 | 2 | 2 | 2 |
| Calcium Hydroxide | 2,0 | 2,25 | 2,5 | 2,75 | 3,0 | 3,25 | 3,5 | 3,75 |
| 1,2-Propylenglycol | ad 100 | | | | | | | |

Vor der Anwendung werden relaxer Creme und Aktivator im Verhältnis 1:1 vermischt.

### b) Verfahren:

Das Haar von Probandinnen wurde mit 20g eines Vorbehandlungsmittels E1 bis E16 behandelt und nach einer Einwrkzeit von 5 Minuten handtuchtrocken frottiert. Anschließend wurden die Haare mit einem Umformungsmittel G behandelt, nach 5 Minuten ausgespült und bei 200°C mit einem Glätteisen geglättet. Folgende Mittel wurden dabei angewendet:

| | | | |
|---|---|---|---|
| E1 → G1 | E5 → G5 | E9 → G9 + G17 | E13 → G13 + G21 |
| E2 → G2 | E6 → G6 | E10 → G10 + G18 | E14 → G14 + G22 |
| E3 → G3 | E7 → G7 | E11 → G11 + G19 | E15 → G15 + G23 |
| E4 → G4 | E8 → G8 | E12 → G12 + G20 | E16 → G16 + G24 |

Zum Vergleich wurden Vorbehandlungsmittel V1, V2, V3 und V4 folgender Zusammensetzung bereitgestellt:

| | **V1** | **V2** |
|---|---|---|
| Dimethicone | 0,15 | - |
| Trideceth-10 | 0,15 | 0,15 |
| Trideceth-5 | 0,1 | 0,1 |
| Parfüm | 0,8 | 0,8 |
| Phenoxyethanol | 0,3 | 0,3 |
| Wasser | ad 100 | |
| | | |

| | **V3** | **V4** |
|---|---|---|
| Dimethicone | 0,1 | - |
| Glycerin | 0,1 | 0,1 |
| Trideceth-10 | 0,1 | 0,1 |
| Trideceth-5 | 0,08 | 0,08 |
| Carbomer (Polyacrylsäure) | 0,1 | 0,1 |
| Parfüm | 0,2 | 0,2 |
| Phenoxyethanol | 0,4 | 0,4 |
| Wasser | ad 100 | |

und analog der vorstehend beschriebenen Verfahren angewendet. Folgende Mittel wurden dabei angewendet:

| | |
|---|---|
| V1 → G1 | V3 → G9 + G17 |
| V2 → G1 | V4 → G9 + G17 |

Die Vorbehandlung mit einem Dimethiconhaltigen Mittel (V1, V3) oder mit einem silikonfreien Mittel (V2, V4) führt zu höherer satischer Aufladung der behandelten Haare. In Kontaktwinkelmessungen konnte zudem eine deutlich gesteigerte Hydrophilität gemessen werden. Die natürliche wasserabweisende Oberflächenfunktion der Haare wurde demnach deutlich stärker beeinträchtigt als bei Vorbehandlung mit erfindungsgemäßen Mitteln, was sich in schlechterem Griff und verschlechterten Kämmbarkeiten zeigte.

## Patentansprüche

1. Verfahren zur Umformung, insbesondere zur Glättung, keratinhaltiger Fasern, insbesondere menschlicher Haare, in welchem
(i) ein Vorbehandlungsmittel, auf die keratinischen Fasern aufgetragen und dort belassen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines 4-morpholinomethyl-substituierten Silikons der Formel (IV) enthält: 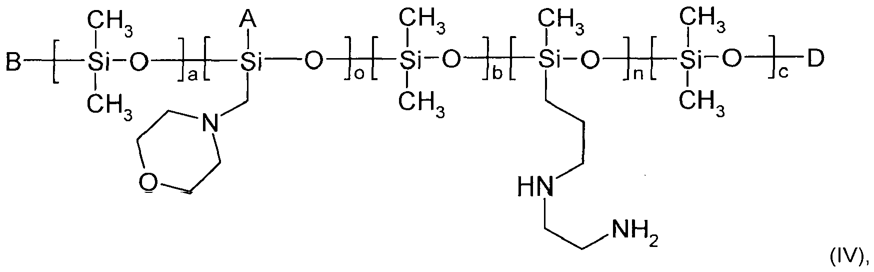 in der
A für eine über ein -O- gebundene Struktureinheit (I) 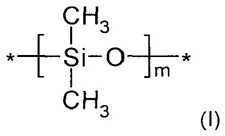 oder einen über ein -O- gebundenen oligomeren oder polymeren Rest enthaltend Struktureinheiten der Formeln (I) oder für-OH steht,
* für eine Bindung zu einer der Struktureinheiten (i), (ii) oder (iii) oder für eine Endgruppe B (Si-gebunden) oder D (O-gebunden) steht,
B für eine Gruppe -OH, -O-Si(CH₃)₃,-O-Si(CH₃)₂OH,-O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen,
mit der Maßgabe, daß mindestens eine der Bedingungen B = -OH bzw. D = -H erfüllt ist.
(ii) die Fasern nach einer Einwirkzeit
- nicht gespült werden,
- gegebenenfalls getrocknet werden,
(iii) die Fasern mit einem alkalischen
Umformungsmittel behandelt werden,
(iv) die Fasern gegebenenfalls gespült werden,
(v) die Fasern gegebenenfalls unter der Einwirkung von Wärme mechanisch verformt werden.

2. Verfahren nach Anspruch 1, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (I) 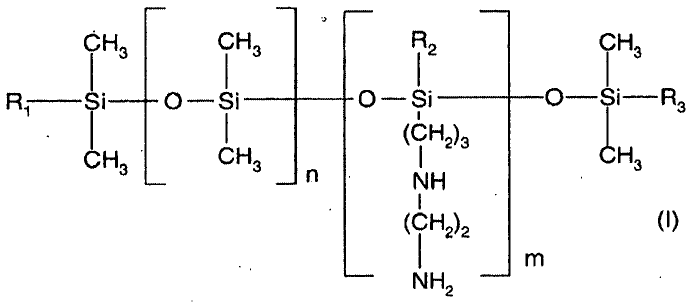 enthält, in der
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

3. Verfahren nach einem der Ansprüche 1 oder 2, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (II) 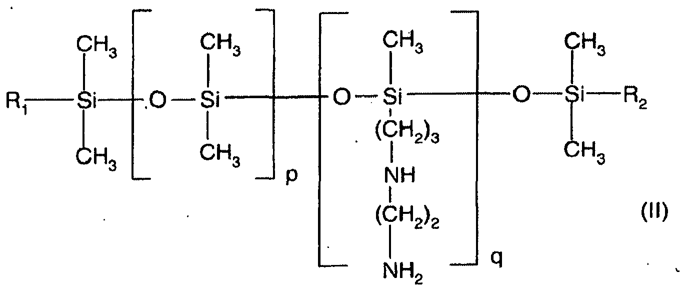 enthält, in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

4. Verfahren nach einem der Ansprüche 1 bis 3, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (III) enthält: 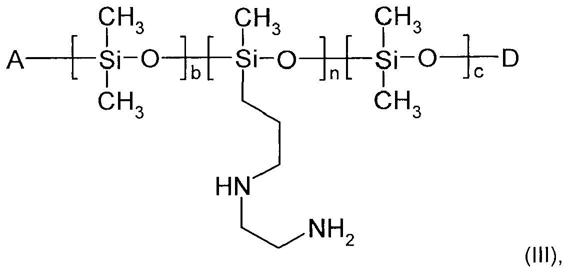 in der
A für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
b, n und c für ganze Zahlen zwischen 0 und 1000 stehen,
mit den Maßgaben
- n > 0 und b + c > 0
- mindestens eine der Bedingungen A = -OH bzw. D = -H ist erfüllt.

5. Verfahren nach einem der Ansprüche 1 bis 4, **dadurch gekennzeichnet, daß** die Fasern in Schritt (ii) getrocknet werden.

6. Verfahren nach einem der Ansprüche 1 bis 5, **dadurch gekennzeichnet, daß** die Fasern in Schritt (iii) einer Behandlung mit einem alkalischen Mittel unterworfen werden.

7. Verfahren nach einem der Ansprüche 1 bis 6, **dadurch gekennzeichnet, daß** die Fasern in Schritt (iii) einer Behandlung mit einer keratinreduzierenden Substanz, der Verformung der Faser und der nachfolgenden Behandlung mit einer oxidierenden Substanz unterworfen werden.

8. Verfahren nach einem der Ansprüche 1 bis 7, **dadurch gekennzeichnet, daß** die Fasern in Schritt (v) einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen werden.

## Claims

1. Method for reshaping in particular for smoothing keratin-containing fibers, in particular human hair in which
(i) a pretreatment agent is applied to the keratinic fibers and left there which contains based on its weight 0.01 to 5% by weight preferably 0.025 to 2.5% by weight, more preferably 0.05 to 1.5% by weight, even more preferably 0.075 to 1% by weight and in particular 0.1 to 0.25% by weight of at least one 4-morpholinomethyl-substituted silicone of formula (IV): 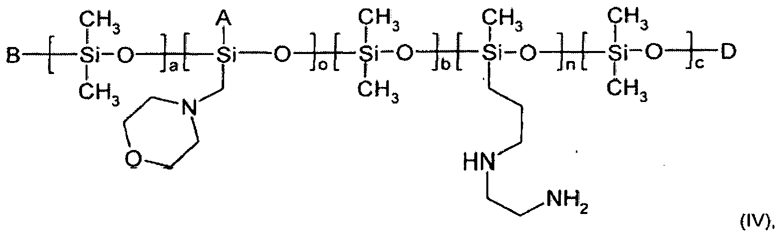 where
A stands for a structure (I) bound by an -O- 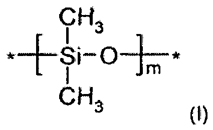 or an oligomeric or polymeric radical bound by an -O- and containing structural units of formula (I), or it stands for -OH,
* stands for a bond to one of the structural units (i), (ii) or (iii) or for an end group B (bound by Si) or D (bound by O),
B stands for a group -OH, -O-Si(CH₃)₃, -O-Si(CH₃)₂OH, -O-Si(CH₃)₂OCH₃,
D stands for a group -H, -Si(CH₃)₃, -Si(CH₃)₂OH, -SI(CH₃)₂OCH₃,
a, b and c stand for integers between 0 and 1000, with the provision that a + b + c > 0
m, n and o stand for numbers between 0 and 1000,
with the provision that at least one of the conditions B = -OH and/or D = -H is satisfied;
(ii) after a treatment time, the fibers
- are not rinsed,
- are optionally dried,
(iii) the fibers are treated with an alkaline remodeling agent,
(iv) the fibers are optionally rinsed,
(v) the fibers are optionally shaped mechanically under the influence of heat.

2. Method according to claim 1, **characterized in that**, in step (i) a pretreatment agent, which contains the following, based on its weight, is applied in step (i): 0.01 to 5% by weight, preferably 0.025 to 2.5% by weight, more preferably 0.05 to 1.5% by weight, even more preferably 0.075 to 1% by weight, and in particular 0.1 to 0.25% by weight of at least one silicone of formula (I): 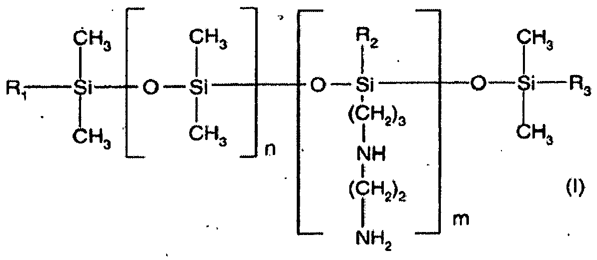 where
- m and n denote numbers that are selected, so that the sum (n + m) is in the range of 1 to 1000,
- n is a number in the range from 0 to 999, and m is a number in the range from 1 to 1000,
- R₁, R₂ and R₃, which are the same or different, denote a hydroxyl group or a C₁₋₄ alkoxy group,
- wherein at least one of the groups R₁ to R₃ denotes a hydroxyl group.

3. Method according to any one of claims 1 or 2, **characterized in that** in step (i) a pretreatment agent is applied, which contains, based on its weight, 0.01 to 5% by weight, preferably 0.025 to 2.5% by weight, more preferably 0.05 to 1.5% by weight, even more preferably 0.075 to 1% by weight and in particular 0.1 to 0.25% by weight of at least one silicone of formula (II) 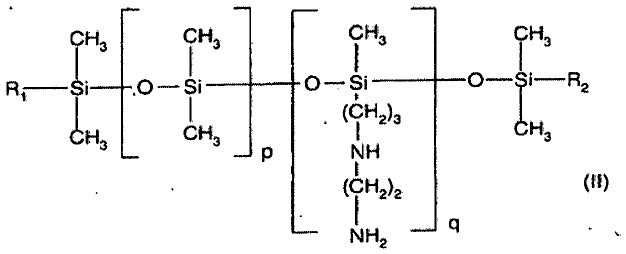 where
- p and q denote numbers that are selected so that the sum (p + q) is in the range of 1 to 1000,
- p is a number in the range from 0 to 999, and q is a number in the range from 1 to 1000,
- R₁ and R₂, which are different, denote a hydroxyl group or a C₁₋₄ alkoxy group, where at least one of the groups R₁ to R₂ denotes a hydroxyl group.

4. Method according to any one of claims 1 to 3, **characterized in that** in step (i) a pretreatment agent is applied, which contains, based on its weight, 0.01 to 5% by weight, preferably 0.025 to 2.5% by weight, more preferably 0.05 to 1.5% by weight, even more preferably 0.075 to 1% by weight and in particular 0.1 to 0.25% by weight of at least one silicone of formula (III): 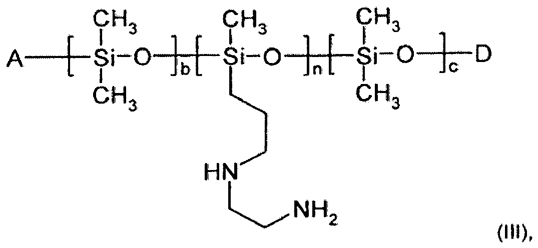 where
A stands for a group -OH, -O-Si(CH₃)₃, -O-Si(CH₃)₂OH, -O-Si(CH₃)₂OCH₃,
D stands for a group -H, -Si(CH₃)₃, -Si(CH₃)₂OH, -SI(CH₃)₂OCH₃,
b, n and c stand for integers between 0 and 1000,
with the provisions that
- n > 0 and b + c> 0,
- at least one of the conditions A = -OH and/or D = -H is satisfied.

5. Method according to any one of claims 1 to 4, **characterized in that** the fibers are dried in step (ii).

6. Method according to any one of claims 1 to 5, **characterized in that** the fibers are subjected to a treatment with an alkaline agent in step (iii).

7. Method according to any one of claims 1 to 6, **characterized in that** the fibers in step (iii) are subjected to a treatment with a keratin-reducing substance, to shaping of the fibers and to a subsequent treatment with an oxidizing substance.

8. Method according to any one of claims 1 to 7, **characterized in that** the fibers are subjected to a heat treatment in step (v) at a temperature of 50°C to 350°C (preferably 80°C to 280°C, especially preferably 100°C to 250°C, more preferably 140°C to 220°C).

## Revendications

1. Procédé de mise en forme, en particulier de lissage, de fibres de kératine, en particulier de cheveux humains, dans lequel
(i) un agent de prétraitement est appliquée sur les fibres de kératine où il est laissé, ledit agent de prétraitement contenant, par rapport à son poids, 0,01 à 5% en poids, de préférence 0,025 à 2,5% en poids, de façon plus préférée 0,05 à 1,5% en poids, encore plus préférablement 0,075 à 1 % en poids et en particulier 0,1 % à 0,25% en poids d'au moins une silicone, substitué avec du 4-morpholinométhyle, de la formule (IV) :
dans laquelle
A représente une unité structure structurelle (I) liée par -O- 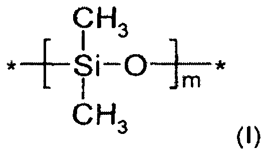
ou un résidu oligomère ou polymère lié par un -O- et contenant des unités structurelles des formules (I), ou -OH,
* représente une liaison à l'une des unités structurelles (i), (ii) ou (iii) ou à un groupe terminal B (lié par Si) ou D (lié par O),
B représente un groupe OH, -O-Si(CH₃)₃, -O-Si(CH₃)₂OH, -O-Si(CH₃)₂OCH₃, 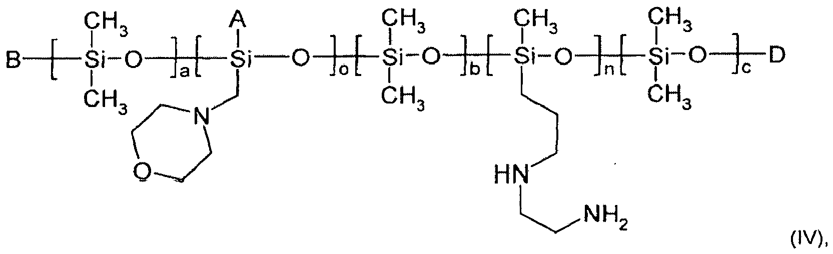
D représente un groupe -H, -Si(CH₃)₃, -Si(CH₃)₂OH, -Si(CH₃)₂OCH₃, a, b et c représentent des nombres entiers compris entre 0 et 1000, avec la condition que a + b + c > 0
m, n et o représentent des nombres entiers compris entre 1 et 1000,
avec la condition que l'une au moins des conditions B = -OH et D = -H soit remplie,
(ii) les fibres au bout un temps d'action
- ne sont pas rincées,
- sont éventuellement séchées,
(iii) les fibres sont traitées avec un agent de mise en forme alcalin,
(iv) les fibres sont éventuellement rincées,
(v) les fibres sont déformées mécaniquement éventuellement sous l'effet de la chaleur.

2. Procédé selon la revendication 1, **caractérisé en ce qu'**un agent de prétraitement est appliqué à l'étape (i), lequel contient, par rapport à son poids, 0,01 à 5% en poids, de préférence 0,025 à 2,5% en poids, de manière plus préférée 0,05 à 1,5% en poids, encore plus préférablement 0,075 à 1% en poids et en particulier 0,1 à 0,25% en poids, d'au moins une silicone de la formule (I) 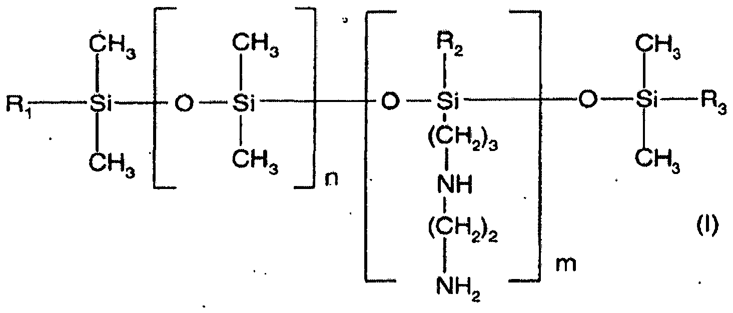 dans laquelle
- m et n sont des nombres qui sont choisis de telle sorte que la somme (n + m) est dans la gamme de 1 à 1000,
- n est un nombre dans la gamme de 0 à 999 et m est un nombre dans la gamme de 1 à 1000,
- R1, R2 et R3, qui sont identiques ou différents, représentent un groupe hydroxy ou un groupe alcoxy en C₁ à C₄,
- l'un au moins des groupes R1 à R3 est un groupe hydroxy.

3. Procédé selon l'une quelconque des revendications 1 ou 2, **caractérisé en ce qu'**un agent de prétraitement est appliqué à l'étape (i), lequel contient, par rapport à son poids, 0,01 à 5% en poids, de préférence 0,025 à 2,5% en poids, de manière plus préférée 0,05 à 1,5% en poids, encore plus préférablement 0,075 à 1% en poids et en particulier 0,1 à 0,25% en poids, d'au moins une silicone de la formule (II) 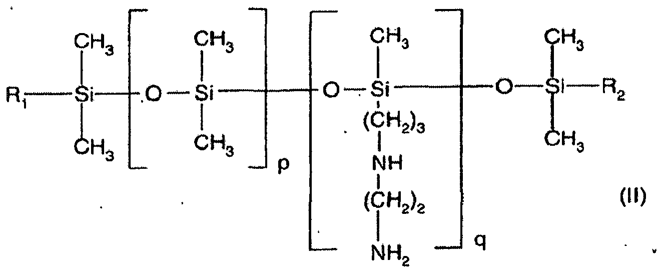 dans laquelle
- p et q sont des nombres qui sont choisis de telle sorte que la somme (p + q) est dans la gamme de 1 à 1000,
- p est un nombre dans la gamme de 0 à 999 et q est un nombre dans la gamme de 1 à 1000,
- R1 et R2, qui sont différents, représentent un groupe hydroxy ou un groupe alcoxy en C₁ à C₄, l'un au moins des groupes R1 à R2 est un groupe hydroxy.

4. Procédé selon l'une quelconque des revendications 1 à 3, **caractérisé en ce qu'**un agent de prétraitement est appliqué à l'étape (i), lequel contient, par rapport à son poids, 0,01 à 5%, de préférence 0,025 à 2,5% en poids, de manière plus préférée 0,05 à 1,5% en poids, encore plus préférablement 0,075 à 1 % en poids, et en particulier 0,1 à 0,25% en poids, d'au moins une silicone de la formule (III) : 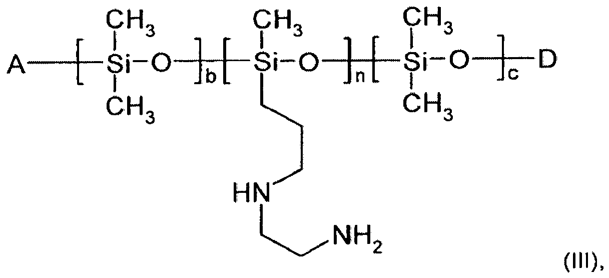 dans laquelle
A représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃, b, c et n sont des nombres entiers compris entre 0 et 1000, avec les conditions
- n > 0 et b + c > 0
- au moins une des conditions A = -OH et D = -H est remplie.

5. Procédé selon l'une quelconque des revendications 1 à 4, **caractérisé en ce que** les fibres sont séchées à l'étape (ii).

6. Procédé selon l'une quelconque des revendications 1 à 5, **caractérisé en ce qu'**à l'étape (iii) les fibres sont soumises à un traitement avec un agent alcalin.

7. Procédé selon l'une quelconque des revendications 1 à 6, **caractérisé en ce qu'**à l'étape (iii) les fibres sont soumises à un traitement avec une substance réduisant la kératine, à une mise en forme de la fibre et à un traitement ultérieur avec une substance oxydante.

8. Procédé selon l'une des revendications 1 à 7, **caractérisé en ce qu'**à l'étape (v) les fibres sont soumises à un traitement thermique à une température de 50°C à 350°C (de préférence de 80°C à 280°C, de manière particulièrement préférée de 100°C à 250°C, plus préférablement de 140°C à 220°C).
